# EUROPEAN PATENT APPLICATION

(11) **EP 2 204 171 A1**
(43) Date of publication of application: **07.07.2010**
(21) Application number: 10159050.3
(22) Date of filing: 06.08.2007
(51) Int. Cl.: A61K 31/00, A61K 31/569, A61P 37/04

(54) **Use of glucocorticoid receptor antagonists for treatment of infectious conditions**

(30) Priority: 08.08.2006 EP 06118587
(62) Divisional of application: 07788248.8
(71) Applicant: N.V. Organon, 5349 AB Oss (NL)
(72) Inventor: Peeters, B.W.M.M., Bernardus, 5340 BH, Oss (NL)
(74) Representative: Broekkamp, Chris L. E.

(57) **Abstract**

The invention provides the use of a glucocorticoid receptor antagonist for the manufacture of a medicament for immune stimulation, such as prevention or treatment of infections or infectious conditions, in an aging mammalian subject, a mammalian subject with low serum DHEAS values, a mammalian subject with a high serum cortisol/DHEAS ratio or a mammalian subject with high neutrophil counts.

In particular, the glucocorticoid receptor antagonist can be chosen from the group consisting of a dibenzopyranyl derivative defined and/or exemplified in US6329534 and WO200116128, mifepristone, and (11β,17β)-11-(1,3-benzodioxol-5-yl)-17-hydroxy-17-(1-propynyl)estra-4,9-dien-3-one.

## Description

The present invention relates to the use of a glucocorticoid receptor (GR) antagonist for an immune stimulant effect in a mammalian subject, and to a pharmaceutical composition containing a GR antagonist. The invention also relates to a method for providing immune stimulation, such as for preventing or treating an infection, or an infectious condition.

It is known that the immune system is influenced by the hypothalamic-pituitary-adrenal (HPA) axis and the sympathetic-adrenal-medullary (SAM) axis. The HPA axis causes the release of glucocorticoid receptor agonists (cortisol in humans) and dehydroepiandrosterone (sulphate) (DHEA(S)), which respectively inhibit and stimulate immune functions. On the other hand, the SAM axis causes the release of catecholamines, which are immunosuppresive.

In healthy, young to middle aged subjects suffering from stress, there is a physiological balance between pro-inflammatory and anti-inflammatory mediators. In the elderly, the immune response is blunted as a result of the decline in several components of the immune system (immune senescence) and a shifting to a chronic pro-inflammatory status (the so-called "inflammaging" effect (Butcher and Lord, (2004) Aging Cell, pp. 151-160). As production of cortisol remains reasonably constant with age, whereas summed levels of DHEA and DHEAS decrease gradually from the third decade, reaching 10-20% of their maximum by the eighth decade, Butcher and Lord (2004, supra) propose a model for age and stress, in which the age-related increase in the ratio of cortisol to DHEAS, combined with an elevated cortisol release during stress, leads to a significant reduction of immunity in aging subjects. This is proposed to explain that aging subjects are far more prone to infections under conditions of stress.

Based on experimental data obtained with white blood cells from elderly hip fracture patients, a supplementation of DHEA and/or DHEAS at times of stress is proposed as a measure to prevent infections (Butcher and Lord (2004, supra); Butcher et al. (2005, Aging Cell 5, pp. 319-324).

The present invention relates to the use of a glucocorticoid receptor antagonist for the manufacture of a medicament for immune stimulation, such as prevention or treatment of infections or infectious conditions, in an aging mammalian subject, such as a human subject. Despite earlier suggestions of immune stimulant effects of counteracting glucocorticosteroid hormones (See Regelson 1990a; 1990b; 1992; Daynes and Araneo 1989; Emilie et al., 1984; Zhang et al., 1998; Morfin et al., US5,763,433; Hampl et al 1998; Lathe, R. et al: WO97/37664; Nakamori et al JP2005213156) and the long-standing availability of a directly acting antagonist of GR receptors, mifepristone (RU 486), there has been no report concerning an immune stimulant effect in normal mammalian subjects obtained with a GR antagonist. Without wishing to be bound by theory, it is believed that the effect in selected subjects as provided by this invention can be explained by the unbalanced immunosuppressive role of the increased cortisol/DHEAS ratio in the aged group in comparison to the balanced influence of cortisol and DHEAS on the immune system in normal subjects. The term 'mammalian subject' includes reference to a human subject, which is a preferred subject for and in the subject-matter provided by the present invention.

If more clarity is needed in the term 'glucocorticoid receptor (GR) antagonist' one can use the definition that a GR antagonist is a compound that can prevent a physiological effect of dexamethasone due to a direct effect of the antagonist on the GR receptor. Therefore, in the reference Saccò et al., 2002 the term 'glucocorticoid receptor antagonist' is used in an unusual manner to describe the influence of DHEA on glucocorticoid action. A direct interaction of a GR antagonist usually implies that the compound has binding affinity for the GR receptor with a pKₐ of at least 6. It can be even more clear to set the lowest limit to the pKₐ in a standard assay for glucocorticoid receptor affinity to 6, 6.5, 7, 7.5, 8, or 9. If the antagonist has remaining agonist effect, as a partial agonist of antagonist, the maximum agonist effect should be less than 50 % of the maximal agonist effect of dexamethasone.

The meaning of the term 'aging subject' or 'aged subject' will be well understood in the context of the use according to this invention. Although it is not linked to an exact lower age limit this general notion refers in the human situation usually to a person of at least 55 years old, but it is more clear with a lowest age limit set at 60, 65, 70 or 75 years.

Preferably, the GR antagonist is chosen from the group consisting of a dibenzopyranyl derivative defined and/or exemplified in US6329534 and WO200116128, mifepristone, and (11β,17β)-11-(1,3-benzodioxol-5-yl)-17-hydroxy-17-(1-propynyl)estra-4,9-dien-3-one, which can be referred to as ORG 34517 and which compound is specifically disclosed in EP 763 541. ORG 34517 is most preferred in view of its high selectivity. Furthermore, ORG 34517 increases both cortisol and DHEA(S) levels. Therefore, ORG 34517 both antagonises the effects of cortisol and increases the effects of DHEA(S), making it the GR antagonist of choice for providing immune stimulation, such as prevention or treatment of infections or infectious conditions, in aging mammalian subjects. The beneficial effects of said GR antagonists, and in particular ORG 34517, may be explained on the basis of their correcting influence on the cortisol/DHEA(S) ratio.

According to a further aspect thereof, the invention provides the use of a glucocorticoid receptor antagonist for the manufacture of a medicament for immune stimulation in an aging mammalian subject under conditions of stress. In the present context, the expression "stress" comprises both physical and psychological stress, either of which can be acute or chronic. Physical stress may be caused by any physical trauma or injury, such as surgery or a fracture. Psychological stress can be caused by emotionally burdening occurrences, such as a bereavement.

According to a further aspect thereof, the invention provides the use of a glucocorticoid receptor antagonist for the manufacture of a medicament for immune stimulation in a mammalian subject having low serum DHEAS values. Especially following a trauma, said values are, in particular in human subjects, usually lower than 6 µM. Use of the GR antagonist for immune stimulation is more preferred in subjects, in particular human subjects, having serum values lower than 4 µM and is most preferred in subjects having serum values lower than 2 µM.

According to a further aspect thereof, the invention provides the use of a glucocorticoid receptor antagonist for the manufacture of a medicament for immune stimulation in a mammalian subject, in particular a human subject, having a high serum cortisol/DHEAS ratio on molar basis. For reasons of clarity this ratio may be further specified as being higher than 0.2. Use of the GR antagonist for immune stimulation is more preferred in such subjects having a serum ratio higher than 0.3 and most preferred in such subjects having a serum ratio higher than 0.4.

According to a further aspect thereof, the invention provides the use of a glucocorticoid receptor antagonist for the manufacture of a medicament for immune stimulation in a mammalian subject, in particular a human subject, having high neutrophil counts. For clarity reasons said counts may be further specified to be higher than 6,5x10⁹ I⁻¹. Use of the GR antagonist for immune stimulation is preferred in such subjects having counts higher than 12x10⁹ I⁻¹ and is most preferred in such subjects having counts higher than 14x10⁹ I⁻¹.

According to a further aspect thereof, the invention provides the use of a glucocorticoid receptor antagonist for the manufacture of a medicament for immune stimulation in a subject having an impaired neutrophil function, such as a reduced superoxide generation (which can be measured in vitro using luminometry). For clarity reasons said reduced superoxide generation may be further specified to amount to a reduction of more than 20%. The use of the GR antagonist for immune stimulation is preferred in such subjects, in particular a human subject, in which the reduction in superoxide generation amounts to more than 40%, and most preferred in such subjects having a reduction amounting to more than 50% of the normal superoxide generation.

Preferably, the GR antagonist is used for the manufacture of a medicament for immune stimulation in a mammalian subject showing both high neutrophil counts and an impaired neutrophil function.

In the context of the invention, the infection or infectious condition can be caused by any of several agents, e.g. by bacteria, by viruses or by fungi. Also in the context of the present invention, the expression "infectious conditions" means silent or subclinical infections as well as conditions not resulting in a manifest infectious disease, but in which at least one parameter associated with an infectious disease, such as the white blood (e.g. neutrophil, basophil or eosinophil) cell counts or the level of some antibodies or some cytokines is higher than normal. Normal values are known to the expert and may be found in standard medical manuals.

Particular uses according to the invention relate to aging subjects suffering from an infection or an infectious condition concomitant to stress resulting from a trauma. The invention particularly relates to uses wherein the subject suffers from the consequences of a hip fracture and/or hip surgery. The invention also relates to uses wherein the subject suffers from an infection or an infectious condition concomitant to psychological stress, particularly acute emotional stress.

Particular infections which can be prevented or treated according to the present invention are upper respiratory tract infections and urinary tract infections.

As aging female subjects, as a result of conditions such as osteoporosis, can be especially prone to the consequences of stress caused by fractures, and as GR antagonists such as RU 486 have an anti-progestagenic action (RU 486 is approved for medical abortions), which makes them unsuitable for use with young or adult females, it is a further advantage of the invention when it is used for a aging female subject. Incidentally, the anti-progestagenic effect of ORG 34517 is merely residual.

The invention also relates to a pharmaceutical composition for immune stimulation, such as prevention or treatment of infections or infectious conditions, in a mammalian subject, chosen from the group comprising an aging mammalian subject, a subject with low DHEAS values, a subject with a high cortisoI/DHEAS ratio and a subject with high neutrophil counts, comprising a therapeutically effective amount of a glucocorticoid receptor antagonist chosen from the group consisting of a dibenzopyranyl derivative defined and/or exemplified in US6329534 and WO200116128, mifepristone, and ORG 34517, the latter being the preferred glucocorticoid receptor antagonist.

Finally, the invention also relates to a method for providing immune stimulation, such as prevention or treatment of infections or infectious conditions, in a mammalian subject chosen from an aging mammalian subject, a subject with low DHEAS values, a subject with a high cortisol/DHEAS ratio and a subject with high neutrophil counts, comprising administering a therapeutically effective amount of a glucocorticoid receptor antagonist chosen from the group consisting of a dibenzopyranyl derivative defined and/or exemplified in US6329534 and WO200116128, mifepristone, and ORG 34517 to a subject in need of such a treatment, ORG 34517 being the preferred glucocorticoid receptor antagonist.

The GR antagonists according to the invention can be administered enterally of parentally, and for humans preferably in a daily dosage of 0,001-10 mg per kg body weight. Mixed with pharmaceutically suitable auxiliaries the GR antagonists may be compressed into solid dosage units, such as pills and tablets, or be processed into capsules or suppositories. By means of pharmaceutically suitable liquids, the GR antagonists can also be applied as an injection preparation in the form of a solution, suspension, emulsion or as a spray, e.g. a nasal spray. For the production of dosage units, e.g. tablets, the use of conventional additives such as fillers, colorants, polymeric binders and the like is contemplated. In general, any pharmaceutically acceptable additive which does not interfere with the function of the active compounds can be used. Suitable carriers with which the compositions can be administered include lactose, starch, cellulose derivatives and the like, or mixtures thereof, used in suitable amounts.

The present invention is illustrated by the following example, which should not be construed as a limitation of the scope of the invention.

### Example.

Clinical observations and tests with white blood cells (in particular neutrophils) obtained from patients and appropriate controls can be used to demonstrate the effect of the invention. The cellular innate immune system, and in particular the neutrophil component thereof, is the first line of defence against bacterial infections, which appear to be the predominant form of infection concomitant to stress. Neutrophils are a type of granulocytes (themselves a group of white blood cells). Their key bactericidal mechanism involves phagocytosis and intracellular killing by release of toxic granular enzymes and the generation of highly toxic reactive oxygen species (such as superoxide, through the enzyme superoxide dismutase). The generation of active oxygen species can be measured very precisely and reproducibly by luminometry.

A major advantage of using luminometric analysis to determine and evaluate the function of neutrophils is that the major immune protective cells of actual patients and controls can be studied under carefully controlled conditions in vitro, in parallel to the in vitro observations.

Neutrophils are isolated from blood (with an anticoagulant) obtained by venipuncture of the forearm of experimental subjects and controls. Following differential centrifugation steps - the last step through "Lymphoprep" produced by Nycomed Inc. - a cell fraction consisting of granulocytes, mainly comprising neutrophils, is obtained. The number and composition of the cell fraction is determined using a Coulter Counter. The neutrophils are stimulated with the oligopeptide FMLP, which mimics bacterial proteins. The light emitted as a consequence of the resulting production of oxygen radicals by the cells is "amplified' by luminol and measured with a luminometer (Berthold). The height of the obtained peaks can be used as an indication of the physiological condition of the neutrophils. Furthermore, the neutrophils can be brought into contact with experimental compounds before the stimulation and measurement, in order to observe the effect on the physiological condition of the neutrophils.

### References

Butcher and Lord, (2004) Aging Cell, pp. 151-160
Butcher et al. (2005), Aging Cell 5, pp. 319-324)
Clark., R.D. et al WO2005/070893: Azadecalin glucocorticoid receptor modulators.
Daynes R.A. and Araneo B. A. Contrasting effects of glucocorticoids on the capacity of T cells to produce the growth factors interleukin 2 and interleukin 4. European J. Immunol 19: 2319, 1989.
Emilie, D. et al., Inhibition of in vitro immunosuppressive effects of glucocorticosteroids by a competitive antagonist RU 486. Immunol. Letters 8: 183-186, 1984.
Hampl, R. and Starka, L.: Abstract with Database accession No EMB-1998070754 on: Dehydroepiandrosterone: 'A fountain of youth' in the light of recent knowledge. Casopis Lekaru Ceskych 1998, Vol 137 pp 8-12.
Lathe, R. et al: WO97/37664: Use of 7-alpha-substituted steroids to treat neuropsychiatric, immune or endocrine disorders.
Morfin., R. US5,763,433: Pharmaceutical compositions containing 2-Beta-hydroxylated 6,7-substituted steroid derivatives, and use thereof. 1998
Nakamori, M. et al JP2005213156 (2005): Abstract of: Composition for use as antistress agent and stress induced infected state improving agent, for preventing/treating infectious disease e.g. cold or influenza, contains Muira Puama and Helianthus annuus dried seeds and/or zikkopi.
Regelson, W. RU 486: How abortion politics have impacted on a potentially useful drug of broad medical application. Perspectives in Biology and Medicine, 35, 3 1002; pp 330-338, 1992.
Regelson W. et al Beyond abortion: RU 486 and the needs of the crisis constituency. JAMA 264: 1026-1027. 1990
Regelson W. Ru 486: A "pro-life" perspective. Medical Aspects of Human sexuality 24 (12): 55-57, 1990.
Saccò, M., Valenti, G., Corvi Mora, P., Wu, F.C.W. and Ray, D.W. DHEA, a selective glucocorticoid receptor antagonist: its role in immune system regulation and metabolism. J. Endocrinol. Invest. 25: (Suppl. To no. 10): 81-82, 2002.
Zhang et al J. Neuro Immunol 92, pp 139-151 (1998)
   EP 763 541.
   US6329534
   W0200116128

## Claims

1. Use of a glucocorticoid receptor antagonist by having binding affinity for the GR receptor for the manufacture of a medicament for immune stimulation in an aging mammalian subject.

2. Use of a glucocorticoid receptor antagonist by having binding affinity for the GR receptor for the manufacture of a medicament for immune stimulation in a mammalian subject with low serum DHEAS values.

3. Use of a glucocorticoid receptor antagonist by having binding affinity for the GR receptor for the manufacture of a medicament for immune stimulation in a mammalian subject with a high serum cortisol/DHEAS ratio.

4. Use of a glucocorticoid receptor antagonist by having binding affinity for the GR receptor for the manufacture of a medicament for immune stimulation in a mammalian subject with high neutrophil counts.

5. Use of according to any one of claims 1-4, for immune stimulation in an aging human subject under conditions of stress and having at least one of a, b or c:
a) a serum DHEAS value lower than 6 µM;
b) a serum cortisol/DHEAS ratio on molar basis higher than 0.2;
c) a neutrophil count higher than 6,5x10⁹ I⁻¹.

6. The use according to any one of claims 1-5, in which the glucocorticoid receptor antagonist is mifepristone or (11β, 17β)-11-(1,3-benzodioxol-5-yl)-17-hydroxy-17-(1-propynyl)estra-4,9-dien-3-one.

7. The use according to claim 6, in which the glucocorticoid receptor antagonist is (11β, 17β)-11-(1,3-benzodioxol-5-yl)-17-hydroxy-17-(1-propynyl)estra-4,9-dien-3-one.

8. The use according to any one of claims 1-7, in which the subject suffers from an infection or an infectious condition concomitant to a hip fracture and/or hip surgery.

9. Method for providing immune stimulation in a mammalian subject chosen from an aging mammalian subject, a subject with low serum DHEAS values, a subject with a high serum cortisol/DHEAS ratio and a subject with high neutrophil counts, comprising administering a therapeutically effective amount of a glucocorticoid receptor antagonist by having binding affinity for the GR receptor to a subject in need thereof.

10. The method according to claim 9, in which the glucocorticoid receptor antagonist is (11β, 17β)-11-(1,3-benzodioxol-5-yl)-17-hydroxy-17-(1-propynyl)estra-4,9-dien-3-one.
